(19) Europäisches Patentamt
European Patent Office
Office européen des brevets

(11) Publication number: **0 073 971**
**B1**

(12) **EUROPEAN PATENT SPECIFICATION**

(45) Date of publication of patent specification: **21.05.86**

(21) Application number: **82107483.8**

(22) Date of filing: **17.08.82**

(51) Int. Cl.⁴: **C 07 D 417/12,**
C 07 D 417/14,
C 07 D 285/10, A 61 K 31/33

(54) **Thiadiazole oxides as gastric antisecretory agents.**

(30) Priority: **24.08.81 US 295447**

(43) Date of publication of application:
**16.03.83 Bulletin 83/11**

(45) Publication of the grant of the patent:
**21.05.86 Bulletin 86/21**

(84) Designated Contracting States:
**AT BE CH DE FR GB IT LI LU NL SE**

(56) References cited:
**EP-A-0 040 696**
**GB-A-2 067 987**

(73) Proprietor: **MERCK & CO. INC.**
**126, East Lincoln Avenue P.O. Box 2000**
**Rahway New Jersey 07065 (US)**

(72) Inventor: **Baldwin, John J.**
**621 Gypsy Hill Circle**
**Gwynedd Valley Pennsylvania 19437 (US)**
Inventor: **Bolhofer, William A.**
**Star Route**
**Frederick Pennsylvania 19435 (US)**
Inventor: **Lumma, William C., Jr.**
**25 Newman Road, M.R. 1**
**Pennsburg Pennsylvania 18073 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 073 971 B1**

**Description**

Background of the invention

Compounds containing a heterocycle connected to a cyanoguanidine moiety through a linear or cyclic connecting group are known in the art as H2 histamine receptor inhibitors and are active compounds for the suppression of gastric acid secretions. See for example U.S. Patent 3,950,333 to Durant *et. al.,* U.S. patent 4,128,658 to Price *et. al.,* and European Patent 3640 to Jones *et. al.*

In addition, Belgian Patent 875,846, as abstracted in Derwent Abstract 79110B/44, discloses compounds wherein a triazole heterocyclic ring is incorporated into compounds similar to those disclosed in Durant *et. al.,* and Jones *et. al.,* in place of the guanidino end group. Further, U.S. Patent 4,025,527 to Durant *et. al.* and European Patent 2930 (Derwent Abstract 53014B/29) disclose substituted guanidines and substituted 1,1-diaminoethylenes in which two nitrogen atoms are each joined to a carbocycle or heterocycle through a linear connecting group.

GB—A—2 067 987 concerns $H_2$-antagonists useful as anti-ulcer agents containing a thiadiazole heterocycle connected by a connecting link, comprising an amine group, to a group, e.g., a phenyl group which may be substituted by alkyl, hydroxy, alkoxy and halogene. There is no pointer to specific complex guanidino thiazolyl compounds.

In addition, Belgian Patent 885,089 discloses diamino thiadiazole oxide compounds wherein both the amino groups are connected to substituted carbo or hetero cyclic groups through linear connecting chains. However, these prior art compounds are distinctly different from and do not suggest those of the present invention in which one of the connecting chains is phenylene.

The thiadiazole oxide compounds of this invention have been found to possess considerable activity in the suppression of gastric acid secretions. Thus, they are useful in peptic ulcer therapy and in other gastric hypersecretory states such as Zollinger-Ellison syndrome, and in protection against drug induced gastric lesions.

Summary of the invention

This invention is directed to diamino thiadiazole oxide compounds wherein both amino groups are connected to a substituted or unsubstituted phenyl or heterocyclic moiety through a linear and a cyclic connecting group and to processes for the preparation of such compounds.

Description of the invention

The compounds of this invention are represented by the following structural formulae:

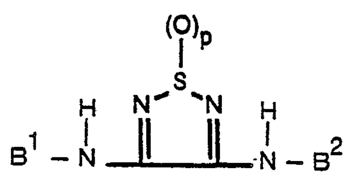

(I)

wherein:

$B^1$ is

R Ⓐ $(CH_2)_nX(CH_2)_m$—;

wherein:

R is hydrogen, $C_{1-5}$alkyl,

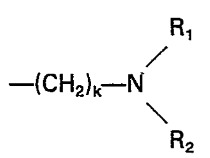

or

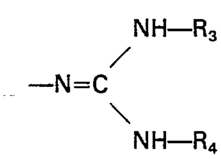

wherein:

$R_1$ and $R_2$ are independently hydrogen, $C_{1-5}$alkyl, $C_{3-7}$cycloalkyl or phenyl-$(C_{1-5})$alkyl or $R_1$ and $R_2$ may be joined to form, along with the nitrogen to which they are attached, a 5- or 6-membered saturated heterocycle, which may also contain an oxygen, sulfur or an N—$R_7$ heteroatom, wherein $R_7$ is hydrogen or alkyl of from 1 to 3 carbon atoms or benzyl, preferably pyrrolidino, piperidino, thiomorpholino, morpholino, or N—$C_{1-3}$alkyl-piperazino;

$R_3$ and $R_4$ are independently hydrogen or $C_{1-5}$alkyl;

n is 0 or 1;

m is 2 to 4;

k is 1 to 4;

X is oxygen, sulfur or methylene;

p is 1 or 2;

Ⓐ is phenylene or a 5-membered heterocycle containing one to three heteroatoms selected from oxygen, sulfur or nitrogen;

provided that when Ⓐ in Formula I above is a 5-membered heterocycle, n is 1; and,

$B^2$ is

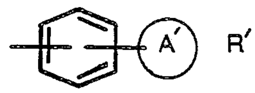

wherein Ⓐ' is thiazole and R' is

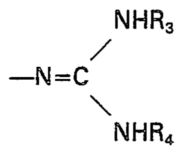

wherein $R_3$ and $R_4$ are defined as above, and the physiologically acceptable salts thereof.

When Ⓐ in Formula I is a furan having the structure

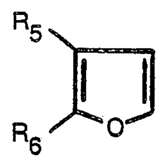

then $R_6$ is the same as R defined above and $R_5$ is $C_{1-5}$alkyl, alkoxyalkyl, alkoxycarbonyl, halo, preferably chlorine or bromine, or aryl, preferably phenyl.

Ⓐ in the foregoing formulae when heterocyclic may be e.g. furan, thiophene, pyrrole, oxazole, oxadiazole, thiadiazole, thiazole, triazole, pyrazole and imidazole.

In the instant invention, unless specified otherwise, the term "loweralkyl" is intended to include those alkyl groups containing from 1 to 5 carbon atoms in either a straight or branched configuration. Examples of such alkyl groups are methyl, ethyl, propyl, *iso*-propyl, butyl, *sec*-butyl and pentyl.

The term "cycloloweralkyl" is intended to include those cycloalkyl groups of from 3 to 7 carbon atoms. Examples of such cycloalkyl groups are cyclopropyl, cyclobutyl, cyclopentyl, cyclohexyl, and cycloheptyl.

In Formula I preferred compounds are realized when p is 1 or 2. Further, such preferred compounds are those wherein Ⓐ in $B^1$ is *m*-phenylene as defined above, n is 0, X is oxygen, m is 3 and R is:

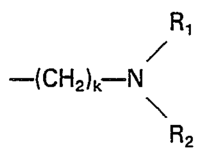

In such cases it is further preferred that k be 1 and $R_1$ and $R_2$ be $C_{1-5}$alkyl, preferably methyl, or joined to form a morpholine, thiomorpholine, piperidine or N-methyl-piperazine ring.

Additionally preferred variations are those wherein Ⓐ is furan, imidazole, thiazole, oxazole, thiophene, triazole, thiadiazole or oxadiazole.

When Ⓐ is heterocyclic it is preferred that n=1, X=sulfur and M=2.

The preferred values of R will depend upon and vary with the definition of Ⓐ.

When Ⓐ is furan, R is preferred to be:

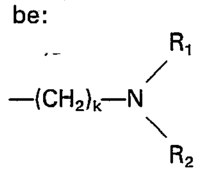

and is attached at the 5-position.

It is further preferred that k=1 and $R_1$ and $R_2$ be hydrogen or $C_{1-5}$alkyl, preferably hydrogen or methyl, or joined to form a morpholine, thiomorpholine, piperidine or N-methyl-piperazine ring.

When Ⓐ is imidazole, R is preferably $C_{1-5}$alkyl, most preferably methyl.

When Ⓐ is thiazole, R is preferably:

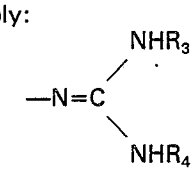

where $R_3$ and $R_4$ are most preferably hydrogen.

In Formula I it is preferred that p is 1 or 2.

It is further preferred that the phenyl ring in $B^2$ be connected to the adjacent groups at its meta position.

It is preferred that in $R^1$

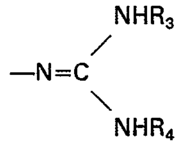

$R_3$ and $R_4$ be hydrogen.

Following is an examplary list of preferred compounds of this invention:

3 - N - [2 - [(4 - methyl - 5 - imidazolyl)methylthio]ethyl]amino - 4 - N - [3 - (2 - guanidino - 4 - thiazolyl)phenyl]amino - 1,2,5 - thiadiazole - 1 - oxide;

3 - N - [2 - [(5 - dimethylaminomethyl - 2 - furanyl)methylthio]ethyl]amino - 4 - N - [3 - (2 - guanidino - 4 - thiazolyl)phenyl]amino - 1,2,5 - thiadiazole - 1 - oxide;

3 - N - [2 - [(2 - guanidino - 4 - thiazolyl)methylthio]ethyl]amino - 4 - N - [3 - (2 - guanidino - 4 - thiazolyl)phenyl]amino - 1,2,5 - thiadiazole - 1 - oxide;

3 - N - [3 - (2 - guanidino - 4 - thiazolyl)phenyl]amino - 4 - N - [3 - [3 - (dimethylaminomethyl)phenoxy]propyl]amino - 1,2,5 - thiadiazole - 1 - oxide;

3 - N - [2 - [(4 - methyl - 5 - imidazolyl)methylthio]ethyl]amino - 4 - N - [3 - [(2 - guanidino - 4 - thiazolyl)phenyl]amino - 1,2,5 - thiadiazole - 1,1 - dioxide;

3 - N - [2 - [(5 - dimethylaminomethyl - 2 - furanyl)methylthio]ethyl]amino - 4 - N - [3 - (2 - guanidino - 4 - thiazolyl)phenyl]amino - 1,2,5 - thiadiazole - 1,1 - dioxide;

3 - N - [2 - [(2 - guanidino - 4 - thiazolyl)methylthio]ethyl]amino - 4 - N - [3 - (2 - guanidino - 4 - thiazolyl)phenyl]amino - 1,2,5 - thiadiazole - 1,1 - dioxide;

3 - N - [3 - [(2 - guanidino - 4 - thiazolyl)phenyl]amino - 4 - N - [3 - [3 - (dimethylaminomethyl)phenoxy]propyl]amino - 1,2,5 - thiadiazole - 1,1 - dioxide.

The compounds of the invention containing basic groups readily form physiologically acceptable salts. Such salts include salts with inorganic acids such as hydrochlorides, hydrobromides, sulfates, nitrates and phosphates. Particularly useful salts of organic acids are formed with aliphatic mono- or dicarboxylic acids. Examples of such salts are acetates, maleates, fumarates, tartrates, citrates, benzoates, succinates and isethionates. The compounds and their salts can also form hydrates and solvates. In addition, the nitrogen atoms in groups R and Ⓐ may also form quaternary salts and N-oxides.

It will also be appreciated by those skilled in the art that the compounds of this invention will have a tautomeric isomerism about the nitrogen atoms adjacent to the thiadiazole ring. Where one or both of the adjacent nitrogen atoms has a hydrogen atom thereon, the *exo*-imino structure can be formed on one or both of such nitrogen atoms as shown in the following structure:

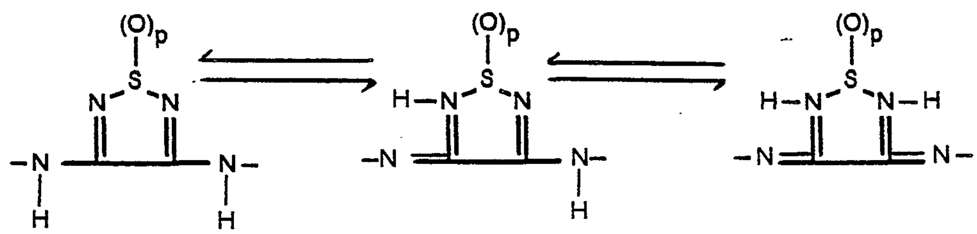

(II)

All of the various tautomeric structures of the instant compounds are intended to be included in this invention.

When R is guanidino, three tautomeric structure variations are possible, and all such tautomers are included in this invention.

In addition, when p is 1, there exists the possibility of stereoisomerism in the instant compounds, corresponding to an R or S absolute configuration at the sulfur atom of the thiadiazole ring. All such stereoisomers are also included within the instant invention.

As stated above, the compounds represented by Formulae I have been found to have pharmacological activity in the animal body as antagonists to certain actions of histamine which are not blocked by "antihistamines" such as mepyramine (N - [(4 - methoxyphenyl)methyl] - N',N' - dimethyl - N - 2 - pyridinyl - 1,2 - ethanediamine).

For example, they have been found to inhibit selectively the histamine-stimulated secretion of gastric acid in the stomach of chronic fistula dogs at doses of from 0.03 to 1.0 mg per kilogram intravenously. Similarly, the action of these compounds is demonstrated by their antagonism to the effects of histamine on other tissues which are not affected by histamine H1 antagonists. An example of such tissue is the isolated guinea-pig right atrium.

These compounds have been found to be up to 50 times more active in animal models than currently marketed gastric antisecretory H2 antihistamines.

The pharmaceutical carrier employed can be, for example, either a solid or liquid. Exemplary of solid carriers are lactose, terra alba, sucrose, talc, gelatin, agar, pectin, acacia, magnesium stearate and stearic acid. Exemplary of liquid carriers are syrup, peanut oil, olive oil and water.

A wide variety of pharmaceutical forms can be employed. Thus, if a solid carrier is used, the preparation can be tableted, placed in a hard gelatin capsule in powder or pellet form, or in the form of a troche or lozenge. The amount of solid carrier will vary widely but preferably will be from about 15 mg to about 0.4 gm. If a liquid carrier is used, the preparation can be in the form of a syrup, emulsion, soft gelatin capsule, sterile injectable liquid such as an ampoule, or an aqueous or nonaqueous liquid suspension.

The pharmaceutical compositions are prepared by conventional techniques involving procedures such as mixing, granulating and compressing or dissolving the ingredients as appropriate to the desired preparation.

The active ingredient will be present in the composition in an effective amount to inhibit histamine gastric acid secretory activity. The route of administration can be orally or parenterally.

Preferably, each daily dosage will contain the active ingredient in an amount of from about 2 mg to about 1000 mg, most preferably from about 40 to about 500 mg given in a single dose or multiple divided doses.

For therapeutic use, the pharmacologically active compounds of the present invention will normally be adminstered as a pharmaceutical composition comprising, as the sole or as an essential active ingredient, at least one such compound in the basic form or in the form of an addition salt with a pharmaceutically acceptable acid and in association with a pharmaceutical carrier therefor. Such addition salts include those mentioned above.

The compounds of this invention can also be employed with other pharmacologically active compounds. Exemplary of such other pharmacological compounds are anticholinergic agents such as propantheline; H1 antihistamines such as mepyramine, pyribenzamine and chlorpheniramine; prostanoids such as prostaglandin E1 and prostaglandin A; histidine decarboxylase inhibitors such as α-fluoromethyl histidine, Brocresin (3 - hydroxy - 4 - bromobenzyloxyamine) and α-hydrazino histidine; antiulcer agents such as carbenoxolone and Vitamin U; antacids such as calcium carbonate preparations and aluminum hydroxide; nitrosation inhibitors such as Vitamin C; antigastrins such as somatostatin; as well as combinations and mixtures thereof.

Advantageously, a pharmaceutical composition will be made up in a dosage unit form appropriate to the desired mode of administration, such as, for example, a tablet, capsule, or injectable solution.

The compounds of the invention are prepared by sequentially reacting a thiadiazole substituted with leaving groups $L_1$ and $L_2$ with appropriate substituted amines. The reaction applies to those compounds wherein p is 1 or 2 and is outlined in the following reaction scheme:

REACTION SCHEME I:

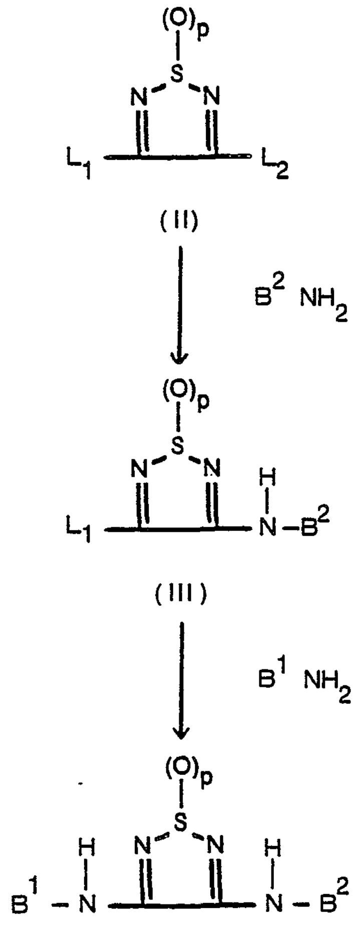


(II)

(III)

(I)

In the foregoing Reaction Scheme, $B^1$ and $B^2$ are as defined above, p is 1 or 2, and $L_1$, and $L_2$ are leaving groups. Appropriate leaving groups for this reaction are exemplified by $C_{1-8}$alkoxy, $C_{1-5}$alkylthio, phenoxy, phenylthio and halogen. Such loweralkoxy and loweralkylthio leaving groups containing from 1 to 5 carbon atoms are in either a straight or branched configuration. Examples of such groups are methoxy, ethoxy, propoxy, *iso*-propoxy, butoxy, *sec*-butoxy, pentoxy, methylthio, ethylthio, propylthio, isopropylthio, phenoxy and phenylthio. The term "halogen" includes the halogen atoms chlorine, fluorine, and bromine.

Preferred leaving groups are methoxy, ethoxy, methylthio, fluorine, chlorine and bromine.

As shown in the foregoing Reaction Scheme, the diaminothiadiazole compounds are prepared by first displacing one of the leaving groups of Compound II with an amine $B^2NH_2$ to afford III followed by displacement of the remaining leaving group by a different amine $B^1NH_2$ to produce the desired compounds. This reaction is carried out on the thiadiazole sulfoxide or sulfone.

With reference to the Reaction Scheme, the reagents are combined, preferably in an aprotic solvent such as tetrahydrofuran, acetonitrile, ethylacetate, ether, and the like, at from −78° to +100°C, preferably from −78° to 100°C. Preferably, 1 equivalent of the amine is added over a period of from 5 minutes to 2 hours. The progress of the reaction can be followed by taking aliquots of the reaction mixture and analyzing them on thin layer chromatography plates. When the reaction is complete, the reaction mixture is allowed to warm and the product is recovered using techniques known to those skilled in the art.

The reaction with the second amine is generally slower than with the first. The reactivity in each case is determined, however, by the particular thiadiazole substrate and the nature of the amine being reacted therewith.

The reaction with the second amine is carried out in a protic or non-protic solvent such as methanol, ethanol, ether, ethylacetate and tetrahydrofuran. The reaction is generally carried out at from 0°C to 100°C and is complete in from 5 minutes to 2 or more days. If the desired reaction temperature is higher than the boiling point of the reaction mixture, the reaction can be carried out in a pressure vessel. Reactions with the thiadiazole sulfones are generally faster than those with the corresponding sulfoxide and, as a result, the

reaction with sulfones are generally complete in shorter times and at lower temperatures than with the sulfoxides. The products are isolated using techniques known to those skilled in the art.

An alternate procedure for the reaction of the first amine with the starting material (II) in which p=2 consists of reacting the trialkylsilyl derivative of the amine $B^2NH_2$ with Compound II in an aprotic solvent at 25°—100°C for about 1 to 24 hours. Required amine trimethylsilyl derivatives are prepared by reacting the amine hydrohalide, preferably hydrochloride, with an excess of the reagent such as hexamethyldisilazane. The reaction is carried out without any solvent from about room temperature to 150°C for from about 1/2 to 24 hours. The thus produced trialkylsilylamine is then reacted with Compound II as described above and the desired product is isolated using techniques known to those skilled in the art.

The thiadiazole starting materials wherein p=2 with two leaving groups thereon are compounds generally known in the art. For example see Carmack *et al. J. Org. Chem. 30* 2743 (1975).

The complex amines which are reacted with the thiadiazole starting material are also compounds known in the art. See for example the Durant *et. al.,* Price *et. al.* and Jones *et. al.* references discussed in the Background of the Invention. Of course, other amine starting materials, similar to those discussed in the foregoing references, can be prepared using the procedures described in such references.

The thiadiazole starting materials wherein p is 1 with two leaving groups thereon are novel compounds. Such compounds are prepared by reacting an appropriately disubstituted oxalimidate or oxalthio-imidate with thionyl chloride to prepare the thiadiazole compounds with two leaving groups as shown below:

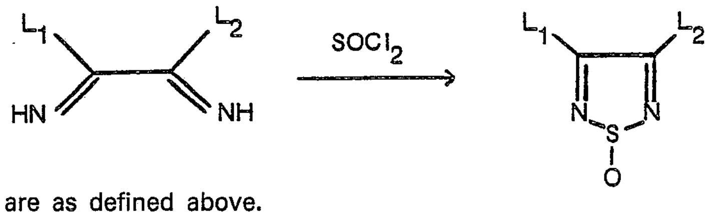


wherein $L_1$ and $L_2$ are as defined above.

The appropriately disubstituted oxalimidate or oxalthioimidate is reacted with thionyl chloride in the presence of a base to neutralize the hydrogen chloride liberated during the course of the reaction. The base may be any organic or inorganic base which does not react with any of the starting materials or products. Generally, tertiary amines such as triethylamine and pyridine or inorganic bases such as alkali metal carbonates or bicarbonates are employed. The reactants are generally dissolved in a suitable solvent such as methylene chloride, chloroform, toluene, ether, tetrahydrofuran and acetonitrile or any other solvent which is non-reactive to the starting materials and products. The reaction is carried out at from −78°C (dry-ice bath) to 100°C or at the reflux temperature of the reaction mixture. The reaction is preferably carried out at from 0°C to room temperature and is generally complete in about from 1/2 to 10 hours. The thionyl chloride is generally used in slight excess although greater amounts of the reagent are not detrimental.

In the following examples which are set forth to further illustrate the invention all temperatures are in degrees Celsius, unless otherwise stated.

Example 1

3 - N - [2 - [(5 - Dimethylaminomethyl - 2 - furanyl)methylthio]ethyl]amino - 4 - N - [3 - (2 - guanidino - 4 - thiazolyl)phenyl]amino - 1,2,5 - thiadiazole - 1 - oxide

A. 4 - N - [3 - (2 - Guanidino - 4′ - thiazolyl)phenyl]amino - 4 - methoxy - 1,2,5 - thiadiazole - 1 - oxide

A solution of 2.33 g (0.010 mol) of 3 - (2 - guanidino - 4 - thiazolyl)benzeneamine in 50 ml of methanol is added to a suspension of 1.94 g (0.010 mol) of 3,4 - bis(methylthio) - 1,2,5 - thiadiazole - 1 - oxide in 20 ml of methanol with stirring. All solids dissolve and the solution is stirred for 120 hours. No change in the reaction mixture is noted by thin layer chromatography during the last 24 hours. The reaction mixture is filtered to give 0.71 g of orange solids which gives only an origin spot on thin layer chromatography. The filtrate is evaporated to dryness under vacuum to give 3.30 g of yellow solid. Nuclear magnetic resonance of the material shows the presence of the product. The material is purified on a column of activity III neutral alumina. Elution with chloroform, followed by 1% methanol/chloroform and 5% methanol/chloroform gives thiadiazole starting material. Elution with 10%, 20% and 50% methanol/chloroform gives 2.32 g (14%) of yellow solid containing product. Recrystallization from acetonitrile gives 1.20 g; m.p. 217—221° (d) of 4 - N - [3 - (2 - guanidino - 4 - thiazolyl)phenyl]amino - 3 - methoxy - 1,2,5 - thiadiazole - 1 - oxide; proton nuclear magnetic resonance is consistent with this structure.

It is noted that during the course of this reaction the solvent (methanol) exchanged with the methylthio group which was not reacted with the amine to prepare the 4-methoxy compound. This does not effect the reaction, since the second amine can react with either the 4-methoxy compound or the 4-methylthio compound.

B. 3 - N - [2 - [(5 - Dimethylaminomethyl - 2 - furanyl)methylthio]ethyl]amino - 4 - N - [3 - (2 - guanidino - 4 - thiazolyl)phenyl]amino - 1,2,5 - thiadiazole - 1 - oxide

The product from part A above is reacted with an equimolar amount of 2 - [(5 -

dimethylaminomethyl - 2 - furanyl)methylthio]ethylamine in acetonitrile and stirred at room temperature for 3 days. The mixture is then concentrated under vacuum and the residue is chromatographed on silica with 5% methanol—95% chloroform. The product fractions are combined and concentrated under vacuum and the title product is isolated by chromatography on silica.

Example 2

3 - N - [3 - (2 - Guanidino - 4 - thiazolyl)phenyl]amino - 4 - N - [3 - [(3 - dimethylaminomethyl)phenoxy]propyl]amino - 1,2,5 - thiadiazole - 1,1 - dioxide

A. 3 - N - [3 - (2 - guanidino - 4 - thiazolyl)phenyl]amino - 4 - ethoxy - 1,2,5 - thiadiazole - 1,1 - dioxide

A solution of 1.17 g (0.005 mol) of 3 - (2 - guanidino - 4 - thiazolyl)benzeneamine in 30 ml of ethanol is added to a slurry of 1.03 g (0.005 mol) of 3,4 - diethoxy - 1,2,5 - thiadiazole - 1,1 - dioxide in 30 ml of ethanol with stirring. All solids dissolve within one minute and a precipitate begins to form within two minutes. The reaction mixture is stirred for 5 hours, at which time the reaction is complete by thin layer chromatography. The precipitate is filtered, dried and recrystallized from ethanol to give 1.35 g of 3 - N - [3 - (2 - guanidino - 4 - thiazolyl)phenyl]amino - 4 - ethoxy - 1,2,5 - thiadiazole - 1,1 - dioxide m.p. 237—239°C.

B. 3 - N - [3 - (2 - Guanidino - 4 - thiazolyl)phenyl]amino - 4 - N - [3 - [(3 - dimethylaminomethyl)phenoxy]propyl]amino - 1,2,5 - thiadiazole - 1,1 - dioxide

The product from part A above is reacted with an equimolar amount of 3 - [(3 - dimethylaminomethyl)phenoxy]propylamine in acetonitrile essentially under the conditions described in Example 1, part B. The title product is isolated by chromatography on silica.

The following preparations are set forth to illustrate the methods which can be employed to obtain the intermediate compounds used in the invention and are not to be construed as being limitative. Unless otherwise indicated, all temperatures are in degrees Celsius.

Preparation 1

3,4-Diethoxy (1,2,5) thiadiazole-1-oxide

To a solution of 441 g (3.06 moles) of diethyl oximidate in 300 ml of methylene chloride and 780 ml (9.63 mol) of pyridine is added dropwise with cooling 195 ml (3.2 mol) of thionyl chloride at 10°±5°C. The reaction mixture is stirred for 2 hours at room temperature. The mixture is partitioned between dilute hydrochloric acid and methylene chloride. The methylene chloride is washed once with dilute hydrochloric and, twice with saturated sodium bicarbonate solution and once with saturated sodium chloride solution.

The organic layer is dried with magnesium sulfate, concentrated to a low volume and diluted with hexane. The crystals are removed by filtration, washed in water and dried *in vacuo* to yield 410 g of 3,4 - diethoxy - 1,2,5 - thiadiazole - 1 - oxide m.p.=74—78°C.

A sample is recrystallized from cyclohexane to yield white crystals m.p.=75—78°C.

Mass spectrum 190 ($M^+$ $C_6H_{10}N_2OS$), 161 ($M^+$ —$C_2H_5$).

IR ($CHCl_3$) 1625 (C=N), 1060 $CM^{-1}$ (SO).

Proton N.M.R. ($CDCl_3$), δ 4.5 (q, J=7Hz, 4H), 1.5 (t, J=7Hz, 6H).

Preparation 2

3,4-Bis-methylthio-1,2,5-thiadiazole-1-oxide

To a solution of 353 g [2.39 moles] of dithiomethyl oximidate in 1500 ml of methylene chloride and 580 ml of pyridine [7.2 moles] is added dropwise 188 ml [2.59 moles] of thionyl chloride at 0±5°C. The temperature of the mixture is raised to room temperature and stirred for 2 hours. The mixture is partitioned between methylene chloride and dilute hydrochloric acid. The organic layer is washed once with dilute hydrochloric acid, twice with saturated sodium bicarbonate and once with saturated sodium chloride solution. The organic layer is dried with magnesium sulfate and concentrated to a low volume and diluted with ether. The crystals are filtered, washed with ether and dried to yield 401 g of 3,4 - bis - methylthio - 1,2,5 - thiadiazole - 1 - oxide m.p.=109—112°C.

Mass spectrum 194 ($M^+$ $C_4H_6OS_3$).

IR ($CHCl_3$) 1625 (C=N), 1050 $CM^1$ (SO).

Proton N.M.R. ($CDCl_3$) 2.7 ppm s.

**Claims for the Contracting States: BE, CH, DE, FR, GB, IT, LI, LU, NL, SE**

1. A compound having the formula:

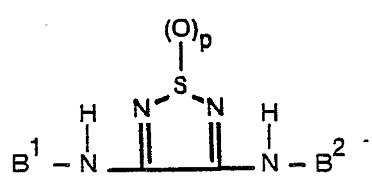

(I)

wherein:

$B^1$ is

R Ⓐ $(CH_2)_nX(CH_2)_m$—;

wherein:

R is hydrogen, $C_{1-5}$alkyl,

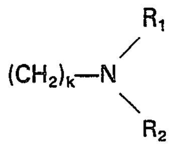

or

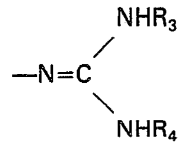

wherein $R_1$ and $R_2$ are independently hydrogen, $C_{1-5}$alkyl, $C_{3-7}$cycloalkyl, or phenyl-($C_{1-5}$)alkyl, or $R_1$ and $R_2$ may be joined to form, along with the nitrogen to which they are attached, a 5- or 6-membered saturated heterocycle, which may also contain an oxygen, sulfur or N—$R_7$ heteroatom, wherein $R_7$ is hydrogen, alkyl of from 1 to 3 carbon atoms or benzyl;

$R_3$ and $R_4$ are independently hydrogen or $C_{1-5}$alkyl;

n is 0 or 1;

m is 2 to 4;

k is 1 to 4;

X is oxygen, sulfur or methylene;

p is 1 or 2;

Ⓐ is phenylene or a 5-membered heterocycle containing one to three heteroatoms selected from oxygen, sulfur or nitrogen;

provided that when Ⓐ is a 5-membered heterocycle, n is 1;

and $B^2$ is

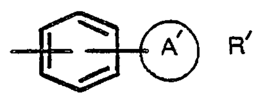

wherein Ⓐ' is thiazole and R' is

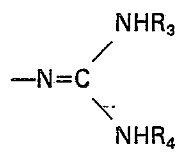

wherein $R_3$ and $R_4$ are defined as above; and, the physiologically acceptable salts thereof.

2. The compound of Claim 1 wherein Ⓐ is independently phenyl, furan, thiophene, pyrrole, oxazole, oxadiazole, thiazole, thiadiazole, triazole or imidazole.

3. The compound of Claim 2 wherein R is

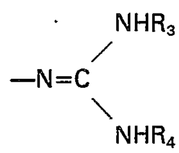

and, Ⓐ is thiazole; n is 1; X is sulfur; m is 2; and $R_3$ and $R_4$ are hydrogen.

4. The compound of Claim 3 wherein Ⓐ is 2,5-furan, R is

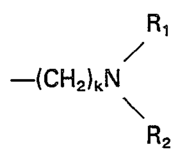

n is 1, X is sulfur, m is 2; and, $R_3$ and $R_4$ are hydrogen.

5. A compound of Claim 1 which is 3 - N - [2 - [(2 - guanidino - 4 - thiazolyl)methylthio]ethyl]amino - 4 - N - [3 - (2 - guanidino - 4 - thiazolyl)phenyl]amino - 1,2,5 - thiadiazole - 1 - oxide.

6. A compound of Claim 1 which is 3 - N - [2 - [(5 - dimethylaminomethyl - 2 - furanyl)methylthio]ethyl]amino - 4 - N - [3 - (2 - guanidino - 4 - thiazolyl)phenyl]amino - 1,2,5 - thiadiazole - 1 - oxide.

7. A compound of Claim 1 which is 3 - N - [3 - (2 - guanidino - 4 - thiazolyl)phenyl]amino - 4 - N - [3 - [3 - (dimethylaminomethyl)phenoxy]propyl]amino - 1,2,5 - thiadiazole - 1 - oxide.

8. A process for the preparation of a compound of Claim 1 which comprises treating a compound having the formula:

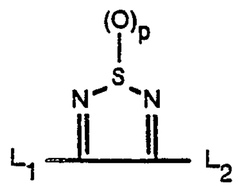

wherein p is 1 or 2 and $L_1$ and $L_2$ are leaving groups, consecutively with the amine compound $B^2$—$NH_2$ followed by the amine compound $B^1$—$NH_2$ wherein $B^1$, and $B^2$ are as defined in Claim 1.

9. A composition useful for suppressing gastric acid secretions in an animal with excess gastric acid secretions which comprises an inert carrier and an anti-secretorily effective amount of a compound of Claim 1.

10. A composition useful for suppressing gastric acid secretions in an animal with excess gastric acid secretions which comprises an inert carrier; an anti-secretorily effective amount of a compound of Claim 1; and, a pharmacologically active compound selected from the group consisting of propantheline (N - methyl - N - (1 - methylethyl) - N - [2 - [(9H - xanthen - 9 - ylcarbonyl)oxy]ethyl] - 2 - propanaminium - derivative, mepyramine (N - [(4 - methoxyphenyl)methyl] - N',N' - dimethyl - N - 2 - pyridinyl - 1,2 - ethanediamine), pyribenzamine (N,N - dimethyl - N' - (phenylmethyl) - N' - 2 - pyridinyl - 1,2 - ethanediamine), chlorpheneramine (γ - (4 - chlorophenyl) - N,N - dimethyl - 2 - pyridine - propanamine), prostaglandins, α-fluormethylhistidine, 3 - hydroxy - 4 - bromobenzyloxy - amine, α-hydrazinohistidine, carbenoxolone (3 - (3 - carboxy - 1 - oxopropoxy) - 11 - oxoolean - 12 - en - 29 - oic acid), Vitamin U (3 - amino - 3 - carboxypropyl) - dimethylsulfonium chloride, calcium carbonate preparations, aluminium hydroxide, Vitamin C (ascorbic acid), somatostatin, as well as combinations thereof.

**Claims for the Contracting State: AT**

1. A process for the preparation of a compound having the formula

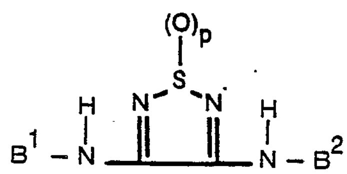

(I)

wherein:
$B^1$ is

$$R(A)(CH_2)_nX(CH_2)_m—;$$

wherein:
R is hydrogen, $C_{1-5}$alkyl,

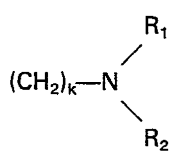

or

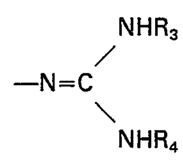

wherein $R_1$ and $R_2$ are independently hydrogen, $C_{1-5}$alkyl, $C_{3-7}$cycloalkyl, or phenyl-($C_{1-5}$)alkyl, or $R_1$ and $R_2$ may be joined to form, along with the nitrogen to which they are attached, a 5- or 6-membered saturated heterocycle, which may also contain an oxygen, sulfur or N—$R_7$ heteroatom, wherein $R_7$ is hydrogen, alkyl of from 1 to 3 carbon atoms or benzyl;

$R_3$ and $R_4$ are independently hydrogen or $C_{1-5}$alkyl;
n is 0 or 1;
m is 2 to 4;
k is 1 to 4;
X is oxygen, sulfur or methylene;
p is 1 or 2;

Ⓐ is phenylene or a 5-membered heterocycle containing one to three heteroatoms selected from oxygen, sulfur or nitrogen;

provided that when Ⓐ is a 5-membered heterocycle, n is 1;

and $B^2$ is

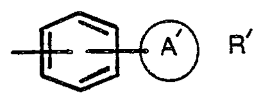

wherein Ⓐ' is thiazole and R' is

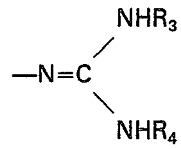

wherein $R_3$ and $R_4$ are defined as above;

and, the physiologically acceptable salts thereof, which comprises treating a compound having the formula:

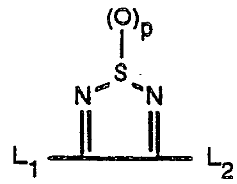

wherein $L_1$ and $L_2$ are leaving groups, consecutively with the amine compound $B^2—NH_2$ followed by the amine compound $B^1—NH_2$.

2. The process of Claim 1 wherein the heterocycle formed when $R_1$ and $R_2$ are joined is a pyrrolidine, piperidine, morpholine, thiomorpholine or piperazine.

3. The process of Claim 1 wherein Ⓐ is independently phenyl, furan, thiophene, pyrrole, oxazole, oxadiazole, thiazole, thiadiazole, triazole, or imidazole.

4. The process of Claim 3 wherein R is

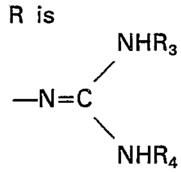

and, Ⓐ is thiazole; n is 1; X is sulfur; m is 2; and $R_3$ and $R_4$ are hydrogen.

5. The process of Claim 4 wherein Ⓐ in $B^1$ is 2,5-furan, R is

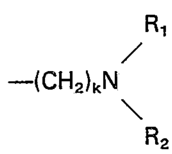

n is 1, X is sulfur, m is 2; and, $R_3$ and $R_4$ are hydrogen.

6. The process of Claim 1 for preparing 3 - N - [2 - [(2 - guanidino - 4 - thiazolyl)methylthio]ethyl]amino - 4 - N - [3 - (2 - guanidino - 4 - thiazolyl)phenyl]amino - 1,2,5 - thiadiazole - 1 - oxide.

7. The process of Claim 1 for preparing 3 - N - [2 - [(5 - dimethylaminomethyl - 2 - furanyl)methylthio]ethyl]amino - 4 - N - [3 - (2 - guanidino - 4 - thiazolyl)phenyl]amino - 1,2,5 - thiadiazole - 1 - oxide.

8. The process of Claim 1 for preparing 3 - N - [3 - (2 - guanidino - 4 - thiazolyl)phenyl]amino - 4 - N - [3 - [3 - (dimethylaminomethyl)phenoxy]propyl]amino - 1,2,5 - thiadiazole - 1 - oxide.

**Patentansprüche für die Vertragsstaaten: BE, CH, DE, FR, GB, IT, LI, LU, NL, SE**

1. Eine Verbindung mit der Formel:

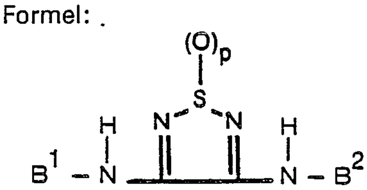 (I)

worin
$B^1$

R Ⓐ $(CH_2)_nX(CH_2)_m$— ist;

wobei:
R Wasserstoff, $C_{1-5}$-Alkyl,

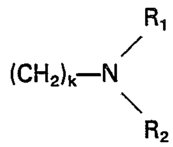 oder 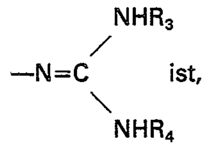 ist,

worin $R_1$ und $R_2$ unabhängig voneinander Wasserstoff, $C_{1-5}$-Alkyl, $C_{3-7}$-Cycloalkyl oder Phenyl-$(C_{1-5})$alkyl sind, oder $R_1$ und $R_2$ zusammen mit dem Stickstoffatom, an das sie gebunden sind, unter Bildung eines 5- oder 6-gliedrigen, gesättigten Heterocyclus, der auch ein Sauerstoff-, Schwefel- oder N—$R_7$-Heteroatom enthalten kann, verbunden sein können, wobei $R_7$ Wasserstoff, Alkyl mit 1 bis 3 Kohlenstoffatomen oder Benzyl ist;

$R_3$ und $R_4$ unabhängig voneinander Wasserstoff oder $C_{1-5}$-Alkyl sind;
n 0 oder 1 ist;
m 2 bis 4 ist;
k 1 bis 4 ist;
X Sauerstoff, Schwefel oder Methylen ist;
p 1 oder 2 ist;
Ⓐ Phenylen oder ein 5-gliedriger Heterocyclus, der 1 bis 3 aus Sauerstoff, Schwefel oder Stickstoff ausgewählte Heteroatome enthält,
mit der Maßgabe ist, daß, falls Ⓐ ein 5-gliedriger Heterocyclus ist, n 1 ist;
und $B^2$

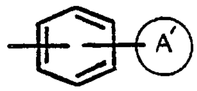 R′ ist,

wobei Ⓐ Thiazol ist und R′

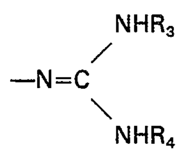 ist,

worin $R_3$ und $R_4$ wie oben definiert sind; und die physiologisch annehmbaren Salze davon.

2. Die Verbindung des Anspruchs 1, worin Ⓐ unabhängig Phenyl, Furan, Thiophen, Pyrrol, Oxazol, Oxadiazol, Thiazol, Thiadiazol, Triazol oder Imidazol ist.

3. Die Verbindung des Anspruchs 2, worin R

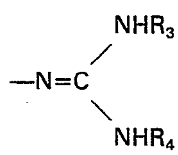

ist, und Ⓐ Thiazol ist; n 1 ist; X Schwefel ist; m 2 ist; und $R_3$ und $R_4$ Wasserstoff sind.

4. Die Verbindung des Anspruchs 3, worin Ⓐ 2,5-Furan ist, R

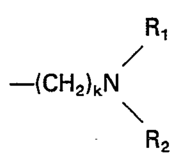 ist,

n 1 ist, X Schwefel ist, m 2 ist; und $R_3$ und $R_4$ Wasserstoff sind.

5. Eine Verbindung des Anspruchs 1, welche 3 - N - [2 - [(2 - Guanidino - 4 - thiazolyl)methylthio]ethyl]amino - 4 - N - [3 - (2 - guanidino - 4 - thiazolyl)phenyl]amino - 1,2,5 - thiadiazol - 1 - oxid ist.

6. Eine Verbindung des Anspruchs 1, welche 3 - N - [2 - [(5 - Dimethylaminomethyl - 2 - furanyl)methylthio]ethyl]amino - 4 - N - [3 - (2 - guanidino - 4 - thiazolyl)phenyl]amino - 1,2,5 - thiadiazol - 1 - oxis ist.

7. Eine Verbindung des Anspruchs 1, welche 3 - N - [3 - (2 - Guanidino - 4 - thiazolyl)phenyl]amino - 4 - N - [3 - [3 - (dimethylaminomethyl)phenoxy]propyl]amino - 1,2,5 - thiadiazol - 1 - oxid ist.

8. Ein Verfahren zur Herstellung einer Verbindung des Anspruchs 1, welches die Behandlung einer Verbindung mit der Formel:

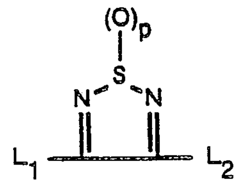

worin p 1 oder 2 ist und $L_1$ und $L_2$ austretende Gruppen sind, nacheinander mit der Aminverbindung $B^2$—$NH_2$ und darauffolgend mit der Aminverbindung $B^1$—$NH_2$, worin $B^1$ und $B^2$ wie in Anspruch 1 definiert sind, umfaßt.

9. Eine Zusammensetzung, die zur Unterdrückung von Magensäureausscheidungen in einem Tier mit übermäßigen Magensäureausscheidungen nützlich ist, welche Zusammensetzung einen inerten Träger und eine antisekretorisch wirksame Menge einer Verbindung des Anspruchs 1 enthält.

10. Eine Zusammensetzung, die zur Unterdrückung von Magensäureausscheidungen in einem Tier mit übermäßigen Magensäureausscheidungen nützlich ist, enthaltend einen inerten Träger; eine antsekretorisch wirksame Menge einer Verbindung des Anspruchs 1; und eine pharmakologisch wirksame Verbindung, ausgewählt aus der Gruppe, die aus Propanthelin (N - Methyl - N - (1 - methylethyl) - N - [2 - [(9H - xanthen - 9 - ylcarbonyl)oxy]ethyl] - 2 - propanaminium - derivat, Mepyramin (N - [(4 - Methoxyphenyl)methyl] - N',N' - dimethyl - N - 2 - pyridinyl - 1,2 - ethandiamin), Pyribenzamin (N,N - Dimethyl - N' - (phenylmethyl) - N' - 2 - pyridinyl - 1,2 - ethandiamin), Chlorpheneramin (γ - (4 - Chlorphenyl) - N,N - dimethyl - 2 - pyridin - propanamin), Prostaglandinen, α-Fluormethylhistidin, 3 - Hydroxy - 4 - brombenzyloxy - amin, α-Hydrazinohistidin, Carbenoxolon (3 - (3 - Carboxy - 1 - oxopropoxy) - 11 - oxoolean - 12 - en - 29 - säure), Vitamin U (3 - Amino - 3 - carboxypropyl) - dimethylsulfoniumchlorid, Calciumcarbonatzubereitungen, Aluminiumhydroxid, Vitamin C (Ascorbinsäure), Somatostatin sowie Kombinationen davon besteht.

**Patentansprüche für den Vertragsstaat: AT**

1. Ein Verfahren zur Herstellung einer Verbindung mit der Formel:

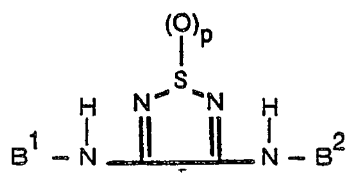 (I)

worin

$B^1$

R(A)$(CH_2)_nX(CH_2)_m$— ist;

wobei

R Wasserstoff, $C_{1-5}$-Alkyl,

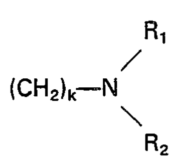 oder 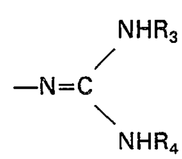 ist,

worin $R_1$ und $R_2$ unabhängig voneinander Wasserstoff, $C_{1-5}$-Alkyl, $C_{3-7}$-Cycloalkyl oder Phenyl-$(C_{1-5})$alkyl sind, oder $R_1$ und $R_2$ zusammen mit dem Stickstoffatom, an das sie gebunden sind, unter Bildung eines 5- oder 6-gliedrigen, gesättigten Heterocyclus, der auch ein Sauerstoff-, Schwefel- oder N—$R_7$-Heteroatom enthalten kann, verbunden sein können, wobei $R_7$ Wasserstoff, Alkyl mit 1 bis 3 Kohlenstoffatomen oder Benzyl ist;

$R_3$ und $R_4$ unabhängig voneinander Wasserstoff oder $C_{1-5}$-Alkyl sind;

n 0 oder 1 ist;

m 2 bis 4 ist;
k 1 bis 4 ist;
X Sauerstoff, Schwefel oder Methylen ist;
p 1 oder 2 ist;
Ⓐ Phenylen oder ein 5-gliedriger Heterocyclus, der 1 bis 3 aus Sauerstoff, Schwefel oder Stickstoff ausgewählte Heteroatome enthält,
mit der Maßgabe ist, daß, falls Ⓐ ein 5-gliedriger Heterocylus ist, n 1 ist;
und $B^2$

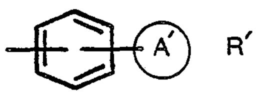
ist,

wobei Ⓐ Thiazol ist und R'

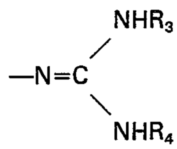
ist,

worin $R_3$ und $R_4$ wie oben definiert sind; und der physiologisch annehmbaren Salze davon, welches die Behandlung einer Verbindung mit der Formel:

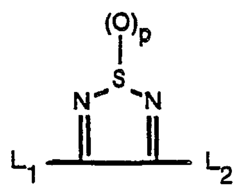

worin $L_1$ und $L_2$ austretende Gruppen sind, nacheinander mit der Aminverbindung $B^2$—$NH_2$ und darauffolgend mit der Aminverbindung $B^1$—$NH_2$, umfaßt.

2. Das Verfahren des Anspruchs 1, worin der Heterocyclus—der gebildet wird, wenn $R_1$ und $R_2$ miteinander verbunden sind—ein Pyrrolidin, Piperidin, Morpholin, Thiomorpholin oder Piperazin ist.

3. Das Verfahren des Anspruchs 1, worin Ⓐ unabhängig Phenyl, Furan, Thiophen, Pyrrol, Oxazol, Oxadiazol, Thiazol, Thiadiazol, Triazol oder Imidazol ist.

4. Das Verfahren des Anspruchs 3, worin R

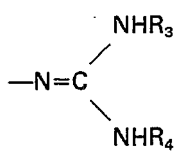

ist, und Ⓐ Thiazol ist; n 1 ist; X Schwefel ist; m 2 ist; und $R_3$ und $R_4$ Wasserstoff sind.

5. Das Verfahren des Anspruchs 4, worin Ⓐ in $B^1$ 2,5-Furan ist, R

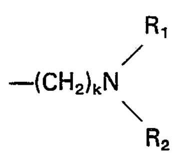
ist,

n 1 ist, X Schwefel ist, m 2 ist; und $R_3$ und $R_4$ Wasserstoff sind.

6. Das Verfahren des Anspruchs 1 zur Herstellung von 3 - N - [2 - [(2 - Guanidino - 4 - thiazolyl)methylthio]ethyl]amino - 4 - N - [3 - (2 - guanidino - 4 - thiazolyl)phenyl]amino - 1,2,5 - thiadiazol - 1 - oxid.

7. Das Verfahren des Anspruchs 1 zur Herstellung von 3 - N - [2 - [(5 - Dimethylaminomethyl - 2 - furanyl)methylthio]ethyl]amino - 4 - N - [3 - (2 - guanidino - 4 - thiazolyl)phenyl]amino - 1,2,5 - thiadiazol - 1 - oxid.

8. Das Verfahren des Anspruchs 1 zur Herstellung von 3 - N - [3 - (2 - Guanidino - 4 - thiazolyl)phenyl]amino - 4 - N - [3 - [3 - (dimethylaminomethyl)phenoxy]propyl]amino - 1,2,5 - thiadiazol - 1 - oxid.

**Revendications pour les Etats Contractants: BE, CH, DE, FR, GB, IT, LI, LU, NL, SE**

1. Composé ayant la formule:

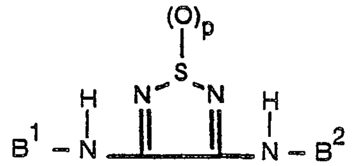 (I)

dans laquelle:

$B^1$ est

$R(A)(CH_2)_nX(CH_2)_m—$;

dans lequel:

R est l'hydrogène ou un alkyle en $C_{1-5}$,

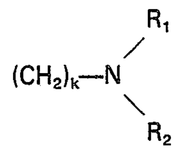 ou 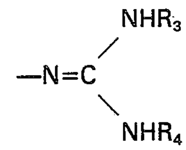

dans lesquels:

$R_1$ et $R_2$ sont indépendamment de l'hydrogène, un alkyle $C_{1-5}$, un cycloalkyle $C_{3-7}$ ou un phényle-alkyle $C_{1-5}$, ou $R_1$ et $R_2$ peuvent être reliés pour former, avec l'azote auquel ils sont liés, un hétérocycle saturé à 5 ou 6 maillons, qui peut aussi contenir un oxygène, du soufre ou un hétéroatome $N—R_7$, dans lequel $R_7$ est l'hydrogène ou un alkyle de 1 à 3 atomes de carbone ou un benzyle;

$R_3$ et $R_4$ sont indépendamment l'hydrogène ou un alkyle $C_{1-5}$;

n est 0 ou 1;

m est 2 à 4;

k est 1 à 4;

X est l'oxygène, le soufre ou le méthylène;

p est 1 ou 2;

Ⓐ est le phénylène ou un hétérocycle à 5 maillons contenant de un à trois hétéroatomes choisis parmi l'oxygène, le soufre ou l'azote;

si, quand Ⓐ dans la formule I est un hétérocycle à 5 chaînons, n'est 1, et $B^2$ est

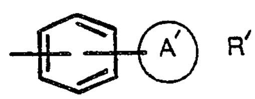

dans lequel Ⓐ' est un thiazole et R' est

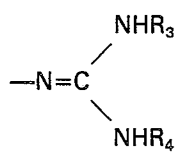

dans lequel $R_3$ et $R_4$ sont définis comme ci-dessus; et, leurs sels convenant physiologiquement.

2. Composé selon la revendication 1, dans lequel Ⓐ est indépendamment un phényle, furanne, thiophène, pyrrole, oxazole, oxadiazole, thiazole, thiadiazole, triazole ou imidazole.

3. Composé selon la revendication 2, dans lequel R est

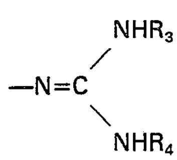

et, Ⓐ est thiazole; n est 1; X est le soufre; m est 2; et $R_3$ et $R_4$ sont l'hydrogène.

4. Composé selon la revendication 3, dans lequel Ⓐ est un 2,5-furannè, R est

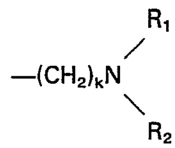

n est 1, X est le soufre, m est 2; et, $R_3$ et $R_4$ sont l'hydrogène.

5. Composé selon la revendication 1 qui est le 3 - N - [2 - [(2 - guanidino - 4 - thiazolyl)méthylthio]éthyl]amino - 4 - N - [3 - (2 - guanidino - 4 - thiazolyl)phényl]amino - 1,2,5 - thiadiazole - 1 - oxyde.

6. Composé selon la revendication 1 qui est le 3 - N - [2 - [(5 - diméthylaminométhyl - 2 - furanyl)méthylthio]éthyl]amino - 4 - N - [3 - (2 - guanidino - 4 - thiazolyl)phényl]amino - 1,2,5 - thiadiazole - 1 - oxyde.

7. Composé selon la revendication 1 qui est le 3 - N - [3 - (2 - guanidino - 4 - thiazolyl)phényl]amino - 4 - N - [3 - [3 - (diméthylaminométhyl)phénoxy]propyl]amino - 1,2,5 - thiadiazole - 1 - oxyde.

8. Procédé de préparation d'un composé selon la revendication 1 qui comprend l'opération de traitement d'un composé ayant la formule:

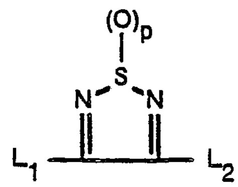

dans laquelle p est 1 ou 2 et $L_1$ et $L_2$ sont des groupes labiles, successivement avec le composé amine $B^2—NH_2$ puis avec le composé amine $B^1—NH_2$ dans lesquels $B^1$ et $B^2$ sont comme définis dans la revendication 1.

9. Composition utilise pour supprimer les sécrétions d'acide gastrique d'un animal ayant des sécrétions excessives d'acide gastrique, qui comprend un véhicule inerte et une quantité efficace d'un composé selon la revendication 1 contre la sécrétion.

10. Une composition utilise pour supprimer les sécrétions d'acide gastrique d'un animal ayant des sécrétions excessives d'acide gastrique qui comprend un véhicule inerte et une quantité efficace d'un composé selon la revendication 1 contre la sécrétion; et un composé pharmacologiquement efficace choisi dans le groupe constitué de propanthéline dérivé de (N - méthyl - N - (1 - méthyléthyl) - N - [2 - [(9H - xanthène - 9 - ylcarbonyloxy]éthyl] - 2 - propanaminium, mépyramine (N - [(4 - méthoxy-phényl)méthyl] - N',N' - diméthyl - N - 2 - pyridinyl - 1,2 - éthanediamine), pyribenzamine (N,N - diméthyl - N' - (phénylméthyl) - N' - 2 - pyridinyl - 1,2 - éthanediamine), chlorphéneramine (γ - (4 - chlorophényl) - N,N - diméthyl - 2 - pyridine - propanamine), prostaglandines, α-fluorométhylhistidine, 3 - hydroxy - 4 - bromobenzyloxy - amine, α-hydrazinohistidine, carbénoxolone acide (3 - (3 - carboxy - 1 - oxopropoxy) - 11 - oxooléane - 12 - ène - 29 - oïque), Vitamine U chlorure de (3 - amino - 3 - carboxypropyl) - diméthyl - sulfonium, préparations de carbonate decalcium, hydroxyde d'aluminium, Vitamine C (acide ascorbique), somatostatine, ainsi que leurs combinaisons.

**Revendications pour l'Etat Contractant: AT**

1. Procédé de préparation d'un composé ayant la formule:

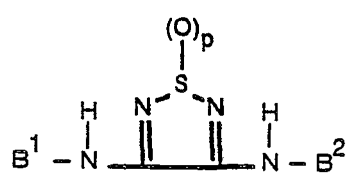

(I)

dans laquelle:

$B^1$ est

$$R{\textcircled{A}}(CH_2)_nX(CH_2)_m—;$$

dans lequel:

R est l'hydrogène, ou un alkyle en $C_{1-5}$,

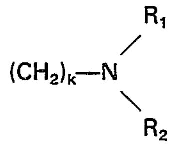

ou

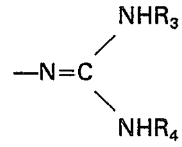

dans lesquels:

$R_1$ et $R_2$ sont indépendamment de l'hydrogène, un alkyle $C_{1-5}$, un cycloalkyle $C_{3-7}$ ou un phényl-alkyle $C_{1-5}$, ou $R_1$ et $R_2$ peuvent être reliés pour former, avec l'azote auquel ils sont liés, un hétérocycle saturé à 5 ou 6 maillons, qui peut aussi contenir un oxygène, un soufre ou un hétéroatome N—$R_7$, dans lequel $R_7$ est l'hydrogène, ou un alkyle de 1 à 3 atomes de carbone ou un benzyle;

$R_3$ et $R_4$ sont indépendamment l'hydrogène ou un alkyle $C_{1-5}$;

n est 0 ou 1;

m est 2 à 4;

k est 1 à 4;

X est l'oxygène, le soufre ou le méthylène;

p est 1 ou 2;

Ⓐ est le phénylène ou un hétérocycle à 5 maillons contenant de un à trois hétéroatomes choisis parmi l'oxygène, le soufre ou l'azote;

si, quand Ⓐ dans la formule I est un hétérocycle à 5 maillons, n est 1, et, $B_2$ est

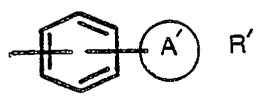

dans lequel Ⓐ' est un thiazole et R' est

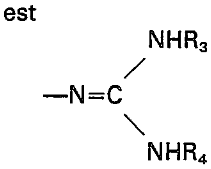

dans lequel $R_3$ et $R_4$ sont définis comme ci-dessus; et, leurs sels physiologiquement acceptables, et qui comprend de traiter un compsé ayant la formule:

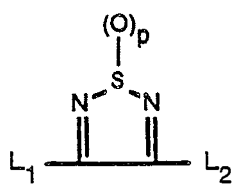

dans laquelle $L_1$ et $L_2$ sont des groupes labiles, successivement avec le composé amine $B^2$—$NH_2$ puis avec le composé amine $B^1$—$NH_2$.

2. Procédé selon la revendication 1, dans lequel l'hétérocycle formé quand $R_1$ et $R_2$ sont reliés est une pyrrolidine, pipéridine, morpholine, thiomorpholine ou pipérazine.

3. Procédé selon la revendication 1, dans lequel Ⓐ est indépendamment un phényle, furanne, thiophène, pyrrole, oxazole, oxadiazole, thiazole, thiadiazole, triazole ou imidazole.

4. Procédé selon la revendication 3, dans lequel R est

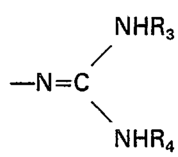

et, Ⓐ est un thiazole; n est 1; X est le soufre; m est 2; et $R_3$ et $R_4$ sont l'hydrogène.

5. Procédé selon la revendication 4, dans lequel Ⓐ dans $B^1$ est un 2,5-furanne, R est

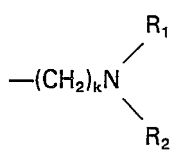

n est 1, X est le soufre, m est 2; et $R_3$ et $R_4$ sont l'hydrogène.

6. Procédé selon la revendication 1 pour préparer le 3 - N - [2 - [(2 - guanidino - 4 - thiazolyl)méthylthio]éthyl]amino - 4 - N - [3 - (2 - guanidino - 4 - thiazolyl)phényl]amino - 1,2,5 - thiadiazole - 1 - oxyde.

7. Procédé selon la revendication 1 pour préparer le 3 - N - [2 - [(5 - diméthylaminométhyl - 2 - furanyl)méthylthio]éthyl]amino - 4 - N - [3 - (2 - guanidino - 4 - thiazolyl)phényl]amino - 1,2,5 - thiadiazole - 1 - oxyde.

8. Procédé selon la revendication 1 pour préparer le 3 - N - [3 - (2 - guanidino - 4 - thiazolyl)phényl]amino - 4 - N - [3 - [3 - (diméthylaminométhyl)phénoxy]propyl]amino - 1,2,5 - thiadiazole - 1 - oxyde.